# EUROPEAN PATENT APPLICATION

(11) **EP 4 494 669 A1**
(43) Date of publication of application: **22.01.2025**
(21) Application number: 23803561.2
(22) Date of filing: 09.05.2023
(51) Int. Cl.: A61M 1/34

(54) **BLOOD PURIFICATION DEVICE**

(30) Priority: 10.05.2022 JP 2022077771
(71) Applicant: Nikkiso Company Limited, Tokyo 150-6022 (JP)
(72) Inventor: FURUHASHI, Tomohiro, Makinohara-shi, Shizuoka 421-0496 (JP); MAKI, Hideto, Makinohara-shi, Shizuoka 421-0496 (JP); OASA, Sho, Makinohara-shi, Shizuoka 421-0496 (JP)
(74) Representative: von Hirschhausen, Helge
(86) International application number: PCT/JP2023/017472
(87) International publication number: WO 2023/219084

(57) **Abstract**

There is provided a blood purification apparatus in which blood remaining in a blood circuit can be smoothly returned to a patient's body even if a blood flow route is clogged or narrowed at the time of blood return, with no need of reconnection of a line for supplying substitution fluid during blood return. A blood purification apparatus includes a physiological-saline supply line (La) connected to an upstream position in a removal-side blood circuit (1a) and through which substitution fluid is allowed to be supplied to a blood circuit (1) at the time of blood return; a pre-substitution line (L2c) and a post-substitution line (L2d) connected to respective downstream positions in the blood circuit (1) and through which the substitution fluid is allowed to be supplied to the blood circuit (1) at the time of blood return, the downstream positions being different from the upstream position; a clogging-identifying unit (7) configured to identify any clogging position of a blood flow route in the blood circuit (1) or a dialyzer (2) with reference to pressures detected by pressure-detecting units (S1 to S3); and a control unit (11) configured to switch from one of a removal-side substitution-fluid supply line and a return-side substitution-fluid supply line to the other at the time of blood return if a clogging position is identified by the clogging-identifying unit (7).

## Description

### Technical Field

The present invention relates to a blood purification apparatus for purifying blood of a patient while causing the blood to extracorporeally circulate.

### Background Art

In general, a blood purification apparatus for giving dialysis treatment includes a removal-side blood circuit and a return-side blood circuit that form a blood circuit for causing blood of a patient to extracorporeally circulate; a blood purifier for purifying the blood extracorporeally circulating through the blood circuit; and an apparatus body provided with various components, such as a blood pump, for performing blood purification treatment with the blood circuit and the blood purifier. The removal-side blood circuit and the return-side blood circuit are each provided with a vascular access catheter or a puncture needle (a removal-side puncture needle or a return-side puncture needle) that is attachable to the distal end thereof.

For example, after the patient is punctured with the removal-side puncture needle and the return-side puncture needle, the blood pump is activated. Thus, blood of the patient extracorporeally circulates through the removal-side blood circuit and the return-side blood circuit. In this process, the blood is purified by the blood purifier. After the blood purification treatment is complete, blood return for returning blood remaining in the blood circuit to the patient is performed, normally. As disclosed by PTL 1 for example, blood return is a process in which blood remaining in the blood circuit is pushed back into the patient's body by supplying substitution fluid, such as physiological saline, into the blood circuit.

### Citation List

### Patent Literature

PTL 1: Japanese Unexamined Patent Application Publication No. 2009-131412

### Summary of Invention

### Technical Problem

In the known blood purification apparatus, if a blood flow route in the blood circuit (particularly in the removal-side blood circuit) is clogged or narrowed because of blood coagulation or the like, the activation of the blood pump at the time of blood return does not cause the blood and the substitution fluid to flow, resulting in difficulty in blood return. In such a case, blood return needs to be stopped, and the remaining blood needs to be disposed of together with the blood circuit; otherwise, the line for supplying the substitution fluid needs to be reconnected to a position downstream of the clogging position so that blood return can be continued.

The present invention has been conceived in view of the above circumstances, and an object of the present invention is to provide a blood purification apparatus in which blood remaining in the blood circuit can be smoothly returned to the patient's body even if the blood flow route is clogged or narrowed at the time of blood return, with no need of reconnection of the line for supplying the substitution fluid during blood return.

### Solution to Problem

A blood purification apparatus according to an embodiment of the present invention includes a blood circuit including a removal-side blood circuit for removing blood from a patient and a return-side blood circuit for returning the blood to the patient, the blood of the patient being caused to extracorporeally circulate through the blood circuit with a blood purifier being connected to the removal-side blood circuit and to the return-side blood circuit, the blood purifier being configured to purify the blood; a blood pump provided to the blood circuit and configured to cause the blood of the patient to extracorporeally circulate through the blood circuit; a removal-side substitution-fluid supply line connected to the blood circuit and through which substitution fluid is allowed to be supplied to the blood circuit at a time of blood return; a return-side substitution-fluid supply line connected to a position of the blood circuit or to the blood purifier and through which the substitution fluid is allowed to be supplied to the blood circuit at the time of blood return, the position of the blood circuit being on a return side relative to a part to which the removal-side substitution-fluid supply line is connected; and a control unit configured to switch the substitution-fluid supply line from one of the removal-side substitution-fluid supply line and the return-side substitution-fluid supply line to an other.

### Advantageous Effects of Invention

According to the present invention, the substitution-fluid supply line is to be switched from one of the removal-side substitution-fluid supply line and the return-side substitution-fluid supply line to the other. Therefore, even if the blood flow route is clogged or narrowed at the time of blood return, blood remaining in the blood circuit can be smoothly returned to the patient's body, with no need of reconnection of the line for supplying the substitution fluid during blood return.

### Brief Description of Drawings

[Fig. 1] Fig. 1 is a schematic diagram of a blood purification apparatus according to a first embodiment of the present invention.
[Fig. 2] Fig. 2 is a schematic diagram of a blood circuit included in the blood purification apparatus.
[Fig. 3] Fig. 3 is a block diagram illustrating how major elements of the blood purification apparatus are connected.
[Fig. 4] Fig. 4 is a flow chart illustrating a process of controlling blood return in the blood purification apparatus.
[Fig. 5] Fig. 5 is a flow chart illustrating another process of blood return.
[Fig. 6] Fig. 6 is a schematic diagram of a blood purification apparatus according to a second embodiment of the present invention.
[Fig. 7] Fig. 7 is a block diagram illustrating how major elements of the blood purification apparatus are connected.
[Fig. 8] Fig. 8 is a flow chart illustrating a process of controlling blood return in the blood purification apparatus.
[Fig. 9] Fig. 9 is a flow chart illustrating another process of blood return.
[Fig. 10] Fig. 10 is a schematic diagram of a blood purification apparatus according to a third embodiment of the present invention.
[Fig. 11] Fig. 11 is a graph illustrating pressure change and flow rate during blood purification treatment performed by the blood purification apparatus.
[Fig. 12] Fig. 12 is a conceptual diagram illustrating how to identify a clogging position by the blood purification apparatus.
[Fig. 13] Fig. 13 is a block diagram illustrating how major elements of the blood purification apparatus are connected.
[Fig. 14] Fig. 14 is a block diagram illustrating how major elements of another blood purification apparatus are connected.
[Fig. 15] Fig. 15 is a schematic diagram of a blood purification apparatus according to a fourth embodiment of the present invention.
[Fig. 16] Fig. 16 is a graph illustrating pressure change and flow rate during blood purification treatment performed by the blood purification apparatus.
[Fig. 17] Fig. 17 is a conceptual diagram illustrating how to identify a clogging position by the blood purification apparatus.
[Fig. 18] Fig. 18 is a block diagram illustrating how major elements of the blood purification apparatus are connected.
[Fig. 19] Fig. 19 is a block diagram illustrating how major elements of another blood purification apparatus are connected.

### Description of Embodiments

Embodiments of the present invention will now be described specifically with reference to the drawings.

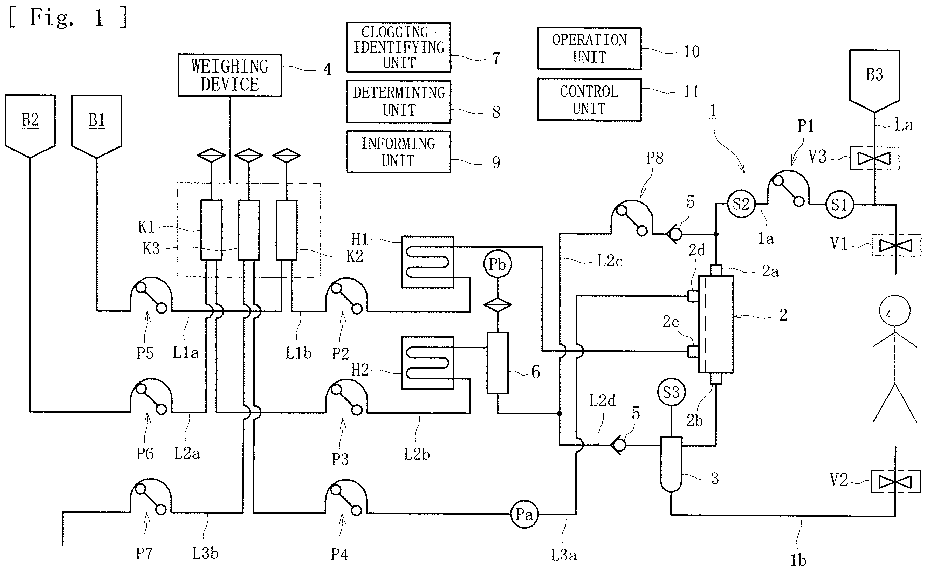

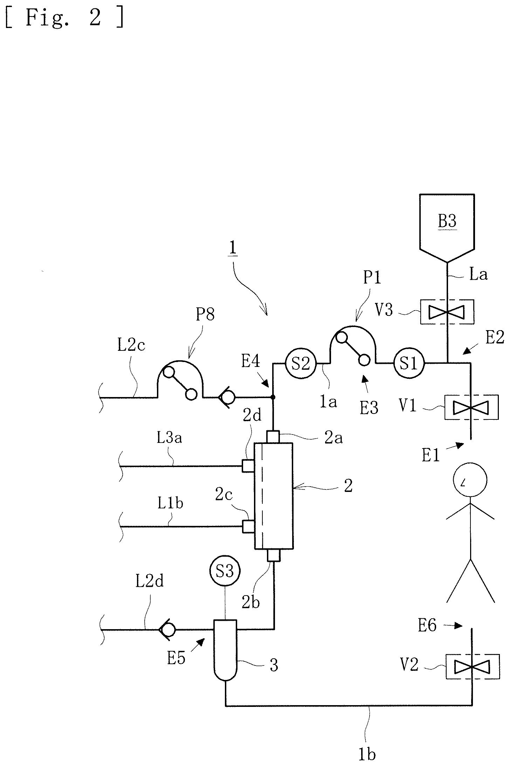

A blood purification apparatus according to a first embodiment is applied to a hemodialysis apparatus (particularly, a dialysis apparatus intended for continuous renal replacement therapy) for purifying blood of a patient while causing the blood to extracorporeally circulate. As illustrated in Figs. 1 and 2, the apparatus includes a blood circuit 1, which includes a removal-side blood circuit 1a and a return-side blood circuit 1b; a dialyzer 2 (blood purifier), which is connected between the removal-side blood circuit 1a and the return-side blood circuit 1b and is configured to purify blood flowing through the blood circuit 1; a first dialysate introduction line L1a and a second dialysate introduction line L1b; a first substitution line L2a and a second substitution line L2b; a physiological-saline supply line La, which serves as a removal-side substitution-fluid supply line; a pre-substitution line L2c and a post-substitution line L2d, which each serve as a return-side substitution-fluid supply line; a first drain-liquid discharge line L3a and a second drain-liquid discharge line L3b; a blood pump P1; a dialysate introduction pump P2; a first substitution pump P3; a drain-liquid discharge pump P4; a dialysate transfer pump P5; a substitution-fluid transfer pump P6; a drain-liquid transfer pump P7; a second substitution pump P8; a reinfusion-fluid temporary chamber K1, a dialysate temporary chamber K2, and a drain-liquid temporary chamber K3; a weighing device 4; a clogging-identifying unit 7; a determining unit 8; an informing unit 9; an operation unit 10; and a control unit 11.

The blood pump P1, the dialysate introduction pump P2, the first substitution pump P3, the drain-liquid discharge pump P4, the dialysate transfer pump P5, the substitution-fluid transfer pump P6, the drain-liquid transfer pump P7, and the second substitution pump P8 according to the present embodiment are each a peristaltic pump configured to deliver liquid by squeezing a flexible tube forming a flow route.

The removal-side blood circuit 1a and the return-side blood circuit 1b are provided at the distal ends thereof with respective connectors, through which a removal-side puncture needle and a return-side puncture needle (not illustrated) are connectable thereto. The removal-side blood circuit 1a and the return-side blood circuit 1b are further provided at distal portions thereof with respective clamp units V1 and V2, which are each an electromagnetic valve or the like and with which respective flow routes are openable and closable as needed. Blood of the patient can be caused to extracorporeally circulate through the blood circuit 1 by opening the clamp units V1 and V2 and activating the blood pump P1 while the patient is punctured with the removal-side puncture needle connected to the distal end of the removal-side blood circuit 1a and the return-side puncture needle connected to the distal end of the return-side blood circuit 1b.

Specifically, when the blood pump P1 is activated while the patient is punctured with the removal-side puncture needle and the return-side puncture needle, the patient's blood flows through the removal-side blood circuit 1a and reaches the dialyzer 2, where the blood is purified. Then, the blood flows through the return-side blood circuit 1b and returns into the patient's body. In this specification, a side on which the puncture needle for removing blood (collecting blood) is provided is referred to as "removal side", and a side on which the puncture needle for returning blood to the patient is provided is referred to as "return side". The "removal side" and the "return side" are not defined by which of the artery and the vein is to be the object of puncture.

The return-side blood circuit 1b is provided with an air-trap chamber 3 at a halfway position thereof. The blood that extracorporeally circulates through the blood circuit 1 undergoes bubble removal at the air-trap chamber 3 and then returns into the patient. As an alternative to the embodiment in which a blood vessel of the patient is punctured with the removal-side puncture needle and the return-side puncture needle, the following may be taken: an embodiment in which a double-lumen catheter is inserted into the subclavian vein or the femoral vein of the patient, an embodiment in which a double-lumen catheter is inserted into a blood vessel in an arm of the patient, or the like.

The dialyzer 2 has a blood introduction port 2a, through which the blood is allowed to be introduced thereinto; a blood delivery port 2b, through which the blood is allowed to be delivered therefrom; a dialysate introduction port 2c, through which dialysate is allowed to be introduced thereinto; a dialysate delivery port 2d, through which the dialysate is allowed to be delivered therefrom; blood flow routes (not illustrated) extending between the blood introduction port 2a and the blood delivery port 2b and through which the blood is allowed to flow; dialysate flow routes (not illustrated) extending between the dialysate introduction port 2c and the dialysate delivery port 2d and through which the dialysate is allowed to flow; and blood purification membranes (not illustrated) separating the blood flow routes from the dialysate flow routes and through which the blood flowing in the blood flow routes can be purified.

More specifically, the dialyzer 2 includes a housing, from which the blood introduction port 2a, the blood delivery port 2b, the dialysate introduction port 2c, and the dialysate delivery port 2d project. The removal-side blood circuit 1a is connected to the blood introduction port 2a. The return-side blood circuit 1b is connected to the blood delivery port 2b. The second dialysate introduction line L1b is connected to the dialysate introduction port 2c. The first drain-liquid discharge line L3a is connected to the dialysate delivery port 2d.

The dialyzer 2 houses a plurality of hollow fiber membranes formed of hollow fibers, which serve as blood purification membranes for purifying the blood. Specifically, spaces inside the respective blood purification membranes formed of the hollow fibers serve as the blood flow routes, and spaces between the housing and the hollow fibers serve as the dialysate flow routes. The blood purification membranes formed of the hollow fiber membranes each have a number of microscopic holes (pores) extending therethrough from the outer surface to the inner surface. Impurities and the like contained in the blood flowing in the blood flow routes can permeate (be filtered) through the hollow fiber membranes into the dialysate flowing in the dialysate flow routes.

The dialysate introduction port 2c is connected to a dialysate bag B1 through the first dialysate introduction line L1a, the dialysate temporary chamber K2, and the second dialysate introduction line L1b. With the activation of the dialysate introduction pump P2 and the dialysate transfer pump P5, dialysate stored in the dialysate bag B1 can be introduced into the dialyzer 2. The dialysate to be introduced into the dialyzer 2 is heated by a heating device H1.

The dialysate delivery port 2d is connected to the first drain-liquid discharge line L3a. With the activation of the drain-liquid discharge pump P4 and the drain-liquid transfer pump P7, drain liquid discharged from the dialyzer 2 can be discharged to the outside of the apparatus through the drain-liquid temporary chamber K3 and the second drain-liquid discharge line L3b. The first drain-liquid discharge line L3a is provided with a pressure sensor Pa, with which the fluid pressure of the drain liquid discharged from the dialyzer 2 can be detected.

A reinfusion-fluid bag B2 stores a predetermined amount of reinfusion fluid (substitution fluid). With the activation of the first substitution pump P3 and the substitution-fluid transfer pump P6, the reinfusion fluid (substitution fluid) can be supplied to the blood circuit 1 through the first substitution line L2a, the reinfusion-fluid temporary chamber K1, and the second substitution line L2b, whereby substitution (pre-substitution or post-substitution) can be performed. The reinfusion fluid (substitution fluid) to be introduced into the blood circuit 1 is heated by a heating device H2 and is subjected to bubble removal at an air-trap chamber 6. The air-trap chamber 6 is provided at the top thereof with a pressure sensor Pb.

More specifically, the second substitution line L2b is connected at the distal end thereof to the pre-substitution line L2c and the post-substitution line L2d. The pre-substitution line L2c is connected to a point of the removal-side blood circuit 1a that is between the blood pump P1 and the dialyzer 2. The post-substitution line L2d is connected to the air-trap chamber 3 provided in the return-side blood circuit 1b. The pre-substitution line L2c and the post-substitution line L2d are each provided with a check valve at a position near the connection thereof to a corresponding one of the removal-side blood circuit 1a and the air-trap chamber 3.

The pre-substitution line L2c is provided with the second substitution pump P8. When the second substitution pump P8 is activated at substantially the same flow as that of the first substitution pump P3, the reinfusion fluid in the reinfusion-fluid temporary chamber K1 can be introduced into the removal-side blood circuit 1a for pre-substitution. On the other hand, when the second substitution pump P8 is stopped while the first substitution pump P3 is in operation, the reinfusion fluid in the reinfusion-fluid temporary chamber K1 can be introduced into the return-side blood circuit 1b for post-substitution.

Furthermore, when the second substitution pump P8 is activated in such a manner as to deliver a lower amount of fluid than the first substitution pump P3, the reinfusion fluid can be introduced into the removal-side blood circuit 1a and the return-side blood circuit 1b for pre- and post-substitution, with a ratio corresponding to the flow rate of the second substitution pump P8. The ratio between the amount of pre-substitution and the amount of post-substitution is changeable by controlling the first substitution pump P3 and the second substitution pump P8 in such a manner as to change the ratio between the amounts of fluid delivery by the first substitution pump P3 and the second substitution pump P8 as needed.

On the other hand, as illustrated in Fig. 2, the removal-side blood circuit 1a of the blood circuit 1 according to the present embodiment is provided with the physiological-saline supply line La (removal-side substitution-fluid supply line), through which substitution fluid (physiological saline, in the present embodiment) is allowed to be supplied to the blood circuit 1 at the time of blood return. The physiological-saline supply line La is connected to an upstream position that is closer to the patient than an installation position E3, where the blood pump P1 is installed (the upstream position is a connection position E2, located between the distal end, E1, of the removal-side blood circuit 1a and the installation position E3 of the blood pump P1). The physiological-saline supply line La is provided with a physiological-saline bag B3, in which a predetermined amount of physiological saline is stored. The flow route of the physiological-saline supply line La is openable and closable by a clamp unit V3.

As illustrated in Fig. 2, the pre-substitution line L2c and the post-substitution line L2d according to the present embodiment are connected to respective downstream positions in the blood circuit 1 that are different from the upstream position (the downstream positions are each located between the installation position E3 of the blood pump P1 and the distal end, E6, of the return-side blood circuit 1b). At the time of blood return, substitution fluid can be supplied to the removal-side blood circuit 1a or the return-side blood circuit 1b. Specifically, the pre-substitution line L2c is connected to a connection position E4, which is located between the installation position E3 of the blood pump P1 and the dialyzer 2; and the post-substitution line L2d is connected to a connection position E5, which is located between the dialyzer 2 and the distal end E6 of the return-side blood circuit 1b (in the present embodiment, the connection position E5 is a position at the air-trap chamber 3).

During blood purification treatment, the clamp units V1 and V2 are open, whereas the clamp unit V3 is closed. Thus, the patient's blood can be subjected to blood purification treatment at the dialyzer 2 while being caused to extracorporeally circulate through the blood circuit 1. Furthermore, if the second substitution pump P8 is selectively activated while the first substitution pump P3 and the substitution-fluid transfer pump P6 are in operation, the reinfusion fluid can be supplied through the pre-substitution line L2c to the removal-side blood circuit 1a for pre-substitution, or through the post-substitution line L2d to the return-side blood circuit 1b for post-substitution.

The blood circuit 1 is further provided at predetermined positions thereof with respective pressure sensors (a first pressure sensor S1, a second pressure sensor S2, and a third pressure sensor S3), each serving as a pressure-detecting unit. Thus, the pressures (fluid pressures) in respective blood flow routes of the blood circuit 1 and the dialyzer 2 are detectable. The first pressure sensor S1 is configured to detect the pressure at the upstream position (the position between the distal end E1 of the removal-side blood circuit 1a and the installation position E3 of the blood pump P1). The second pressure sensor S2 and the third pressure sensor S3 are configured to detect the pressures on the upstream side and the downstream side, respectively, of the dialyzer 2 at the respective downstream positions (positions between the installation position E3 of the blood pump P1 and the distal end E6 of the return-side blood circuit 1b).

Specifically, the first pressure sensor S1 is provided at a position between the connection position E2 of the physiological-saline supply line La and the installation position E3 of the blood pump P1, the second pressure sensor S2 is provided at a position between the installation position E3 of the blood pump P1 and the dialyzer 2, and the third pressure sensor S3 is provided at a position (the air-trap chamber 3) between the dialyzer 2 and the distal end E6 of the return-side blood circuit 1b. Thus, the pressure sensors S1 to S3 are capable of detecting the pressures (fluid pressures) at the respective positions of the blood flow route. Note that a venous pressure sensor configured to detect the pressure of the air layer in the air-trap chamber 3 is utilized as the third pressure sensor S3 to detect the fluid pressure.

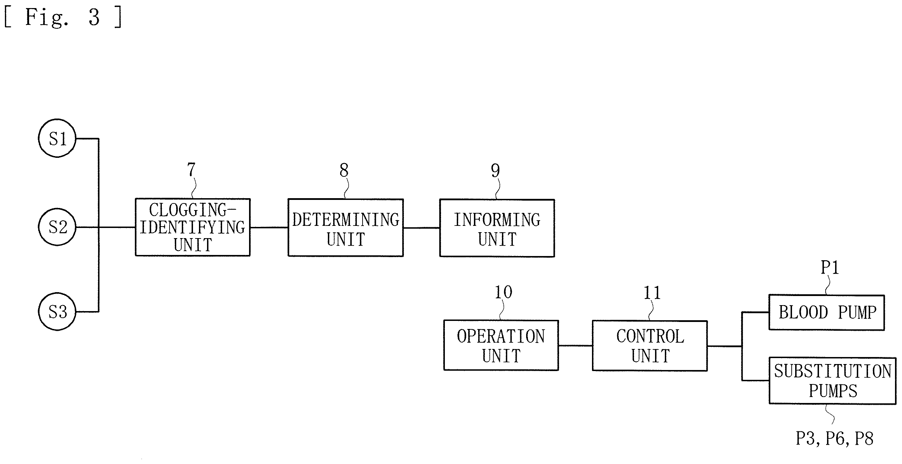

Referring to Fig. 3, the clogging-identifying unit 7 is an arithmetic unit, such as a microcomputer, electrically connected to the first pressure sensor S1, the second pressure sensor S2, and the third pressure sensor S3 and is configured to identify any clogging position of the blood flow route in the blood circuit 1 or the dialyzer 2 with reference to the pressures detected by the first pressure sensor S1, the second pressure sensor S2, and the third pressure sensor S3. The identification of any clogging position by the clogging-identifying unit 7 may be performed either during blood purification treatment or during blood return.

For example, if the value detected by the first pressure sensor S1 increases but the value detected by the second pressure sensor S2 decreases, it can be identified that clogging or narrowing is occurring between the first pressure sensor S1 and the second pressure sensor S2. On the other hand, if the value detected by the second pressure sensor S2 increases but the value detected by the third pressure sensor S3 is normal, it can be identified that clogging or narrowing is occurring between the second pressure sensor S2 and the air-trap chamber 3. If the values detected by the first to third pressure sensors S1 to S3 are all normal, it can be identified that neither clogging nor narrowing is occurring in the blood circuit 1.

Referring to Fig. 3, the determining unit 8 is an arithmetic unit, such as a microcomputer, electrically connected to the clogging-identifying unit 7. If a clogging position is identified by the clogging-identifying unit 7, the determining unit 8 determines to switch the line for supplying the substitution fluid (reinfusion fluid) at the time of blood return from one of the removal-side substitution-fluid supply line (physiological-saline supply line La) and the return-side substitution-fluid supply line (pre-substitution line L2c and post-substitution line L2d) to the other in correspondence with the clogging position.

For example, if it is identified by the clogging-identifying unit 7 that clogging or narrowing is occurring between the connection position E2 of the physiological-saline supply line La and the installation position E3 of the blood pump P1, the determining unit 8 determines to switch from the physiological-saline supply line La to the pre-substitution line L2c to supply the substitution fluid. On the other hand, if it is identified by the clogging-identifying unit 7 that clogging or narrowing is occurring between the connection position E4 of the pre-substitution line L2c and the dialyzer 2, the determining unit 8 determines to switch from the physiological-saline supply line La or the pre-substitution line L2c to the post-substitution line L2d to supply the substitution fluid.

The informing unit 9 is an output device electrically connected to the determining unit 8. If it is determined by the determining unit 8 to switch the substitution-fluid supply lines, the informing unit 9 provides information to switch the substitution-fluid supply lines at the time of blood return. The informing unit 9 is any of the following, for example: a display device, such as a monitor, configured to display an image that prompts switching; an output device, such as a speaker, configured to output a sound that prompts switching; an illumination device, such as an LED, configured to emit light or blink in such a manner as to prompt switching; and the like.

For example, at the time of blood return, if it is determined by the determining unit 8 to switch from the physiological-saline supply line La to the pre-substitution line L2c to supply the substitution fluid, the informing unit 9 provides information to prompt an operation of supplying the substitution fluid (reinfusion fluid) through the pre-substitution line L2c instead of supplying the substitution fluid (physiological saline) through the physiological-saline supply line La. On the other hand, if it is determined by the determining unit 8 to switch from the physiological-saline supply line La or the pre-substitution line L2c to the post-substitution line L2d to supply the substitution fluid, the informing unit 9 provides information to prompt an operation of supplying the substitution fluid through the post-substitution line L2d instead of supplying the substitution fluid through the physiological-saline supply line La or the pre-substitution line L2c.

At the time of blood return, if the information to switch the substitution-fluid supply lines is provided by the informing unit 9, an operation for the switching is to be performed on the operation unit 10. For example, an operation of opening or closing the clamp unit V1 and an operation of activating or stopping relevant pumps can be performed on the operation unit 10. More specifically, the supply of the substitution fluid (physiological saline) through the physiological-saline supply line La may be switched to the supply of the substitution fluid (reinfusion fluid) through the pre-substitution line L2c, by stopping the operation of the blood pump P1 with the clamp unit V1 closed and activating the second substitution pump P8 with the first substitution pump P3 and the substitution-fluid transfer pump P6 being in operation.

On the other hand, the supply of the substitution fluid (reinfusion fluid) through the pre-substitution line L2c may be switched to the supply of the substitution fluid (reinfusion fluid) through the post-substitution line L2d, by keeping the clamp unit V1 closed and the blood pump P1 stopped and stopping the second substitution pump P8 with the first substitution pump P3 and the substitution-fluid transfer pump P6 being in operation. Furthermore, the supply of the substitution fluid (physiological saline) through the physiological-saline supply line La may be switched to the supply of the substitution fluid (reinfusion fluid) through the post-substitution line L2d, by stopping the operation of the blood pump P1 with the clamp unit V1 closed and stopping the second substitution pump P8 with the first substitution pump P3 and the substitution-fluid transfer pump P6 being in operation.

The control unit 11 is configured to switch the substitution-fluid supply line from one of the removal-side substitution-fluid supply line and the return-side substitution-fluid supply line to the other. Specifically, referring to Fig. 3, the control unit 11 according to the present embodiment is a microcomputer or the like electrically connected to each of the operation unit 10, the blood pump P1, the first substitution pump P3, the substitution-fluid transfer pump P6, and the second substitution pump P8. If a predetermined operation is performed by the operator in response to the information provided by the informing unit 9, the control unit 11 switches the substitution-fluid supply line from one of the removal-side substitution-fluid supply line (physiological-saline supply line) and the return-side substitution-fluid supply line (pre-substitution line L2c or post-substitution line L2d) to the other. The operation unit 10 may be electrically connected to all of or some of relevant elements including, for example, the clogging-identifying unit 7, the determining unit 8, and the informing unit 9. Any configuration for electrical connections may be employed.

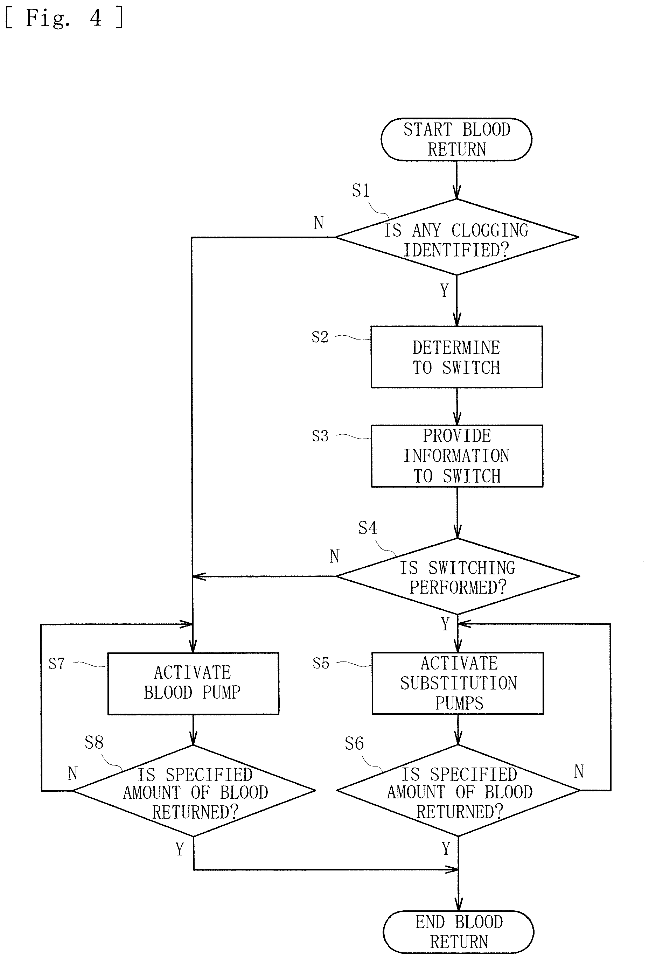

Now, a process of controlling blood return in the blood purification apparatus according to the first embodiment will be described with reference to a flow chart illustrated in Fig. 4.

After the completion of a blood purification treatment, blood return is started. Accordingly, the supply of the dialysate to the dialyzer 2 is stopped, and the blood pump P1 is stopped with the clamp unit V3 open. Thus, the physiological saline in the physiological-saline bag B3 is supplied to the blood circuit 1. With the self-weight of the physiological saline generated by the head difference, the blood in the flow route between the distal end E1 of the removal-side blood circuit 1a and the connection position E2 is substituted for by the substitution fluid, and the blood thus substituted for is returned to the patient.

Subsequently, the clamp unit V1 is closed, and the following process is performed to return the blood in the blood circuit 1 from the connection position E2 to the distal end E6 of the return-side blood circuit 1b. Specifically, in S1, it is checked whether any clogging position is identified by the clogging-identifying unit 7. If it is determined that no clogging position is identified (neither clogging nor narrowing is detected), the blood pump P1 is activated in S7. Then, in S8, whether a specified amount of returned blood is reached is checked. In this step, the first substitution pump P3, the substitution-fluid transfer pump P6, and the second substitution pump P8 are kept stopped. Furthermore, the blood pump P1 is kept in operation until the specified amount of returned blood is reached. If it is determined that the specified amount is reached, blood return is ended.

On the other hand, if it is determined in S1 that a clogging position is identified, the determining unit 8 determines in S2 to switch the substitution-fluid supply lines. For example, if it is identified that clogging or narrowing is occurring between the distal end E1 of the removal-side blood circuit 1a and the installation position E3 of the blood pump P1, the determining unit 8 determines to switch from the physiological-saline supply line La to the pre-substitution line L2c to supply the substitution fluid. On the other hand, if is identified by the clogging-identifying unit 7 that clogging or narrowing is occurring between the installation position E3 of the blood pump P1 and the dialyzer 2, the determining unit 8 determines to switch from the physiological-saline supply line La or the pre-substitution line L2c to the post-substitution line L2d to supply the substitution fluid.

After information to switch the substitution-fluid supply lines is provided in S3 by the informing unit 9, it is checked in S4 whether a switching operation according to the information provided by the informing unit 9 is performed. If it is determined in S4 that the switching operation according to the information provided by the informing unit 9 is performed by the operator, the process proceeds to S5, in which the control unit 11 activates the first substitution pump P3 and the substitution-fluid transfer pump P6 with the blood pump P1 kept stopped, in accordance with the switching operation performed on the operation unit 10. In the switching operation, an operation of closing the clamp unit V3 is also performed.

In this step, if the substitution fluid is to be supplied through the pre-substitution line L2c, the control unit 11 activates the first substitution pump P3 and the substitution-fluid transfer pump P6 with the blood pump P1 stopped, and activates the second substitution pump P8 (thus, the control unit 11 changes the states of operations of the blood pump P1, the first substitution pump P3, the substitution-fluid transfer pump P6, and the second substitution pump P8, thereby switching the substitution-fluid supply line from the physiological-saline supply line La to the pre-substitution line L2c). On the other hand, if the substitution fluid is to be supplied through the post-substitution line L2d, the control unit 11 activates the first substitution pump P3 and the substitution-fluid transfer pump P6 with the blood pump P1 stopped, but keeps the second substitution pump P8 stopped (as with the above case, the control unit 11 changes the states of operations of the blood pump P1, the first substitution pump P3, the substitution-fluid transfer pump P6, and the second substitution pump P8, thereby switching the substitution-fluid supply line from the physiological-saline supply line or the pre-substitution line L2c to the post-substitution line L2d).

After the substitution pumps are activated in S5 (after the first substitution pump P3 and the substitution-fluid transfer pump P6 are activated and the second substitution pump P8 is selectively activated), it is checked in S6 whether a specified amount of returned blood is reached. The substitution pumps are kept in operation (the first substitution pump P3 and the substitution-fluid transfer pump P6 are kept in operation and the second substitution pump P8 is selectively kept in operation) until the specified amount of returned blood is reached. If it is determined that the specified amount is reached, blood return is ended. In this process of blood return, the identification of any clogging position by the clogging-identifying unit 7 can be performed at the last phase of the blood purification treatment.

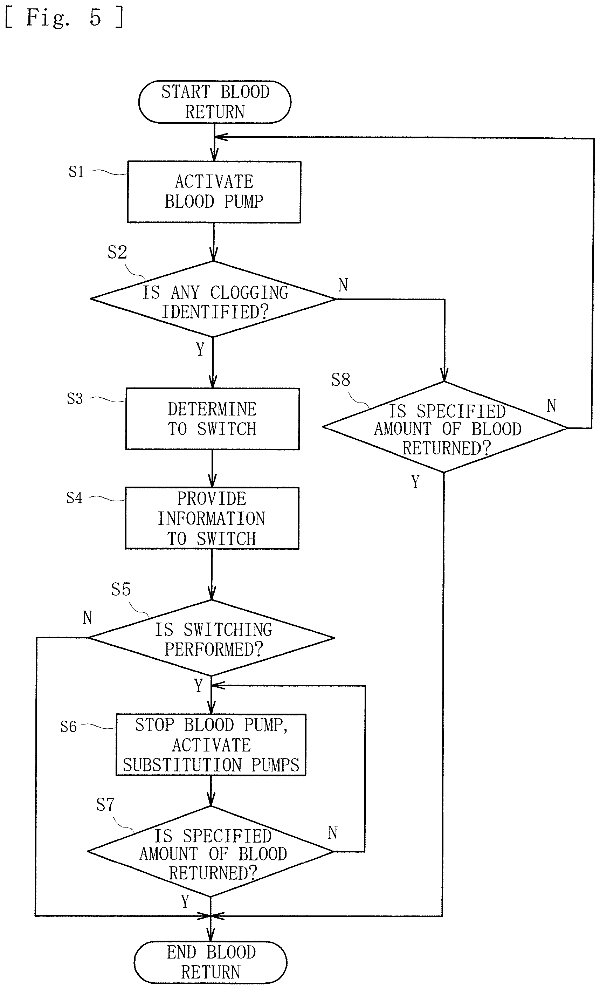

Now, another process of controlling blood return in the blood purification apparatus according to the first embodiment will be described with reference to a flow chart illustrated in Fig. 5.

After the completion of a blood purification treatment, blood return is started. Accordingly, the supply of the dialysate to the dialyzer 2 is stopped, and the blood pump P1 is stopped with the clamp unit V3 open. Thus, the physiological saline in the physiological-saline bag B3 is supplied to the blood circuit 1. With the self-weight of the physiological saline generated by the head difference, the blood in the flow route between the distal end E1 of the removal-side blood circuit 1a and the connection position E2 is substituted for by the substitution fluid, and the blood thus substituted for is returned to the patient.

Subsequently, the clamp unit V1 is closed, and the following process is performed to return the blood in the blood circuit 1 from the connection position E2 to the distal end E6 of the return-side blood circuit 1b. Specifically, in S1, the blood pump P1 is activated. Then, in S2, it is checked whether any clogging position is identified by the clogging-identifying unit 7. If it is determined that no clogging position is identified (neither clogging nor narrowing is detected), it is checked in S8 whether a specified amount of returned blood is reached. In this step, the first substitution pump P3, the substitution-fluid transfer pump P6, and the second substitution pump P8 are kept stopped. Furthermore, the blood pump P1 is kept in operation until the specified amount of returned blood is reached. If it is determined that the specified amount is reached, blood return is ended.

On the other hand, if it is determined in S2 that a clogging position is identified, the determining unit 8 determines in S3 to switch the substitution-fluid supply lines. For example, if it is identified that clogging or narrowing is occurring between the connection position E2 of the physiological-saline supply line La and the installation position E3 of the blood pump P1, the determining unit 8 determines to switch from the physiological-saline supply line La to the pre-substitution line L2c to supply the substitution fluid. On the other hand, if it is identified by the clogging-identifying unit 7 that clogging or narrowing is occurring between the connection position E4 of the pre-substitution line L2c and the dialyzer 2, the determining unit 8 determines to switch from the physiological-saline supply line La or the pre-substitution line L2c to the post-substitution line L2d to supply the substitution fluid.

After information to switch the substitution-fluid supply lines is provided in S4 by the informing unit 9, it is checked in S5 whether a switching operation according to the information provided by the informing unit 9 is performed. If it is determined in S5 that the switching operation according to the information provided by the informing unit 9 is performed by the operator, the process proceeds to S6, in which the control unit 11 stops the blood pump P1 but activates the first substitution pump P3 and the substitution-fluid transfer pump P6, in accordance with the switching operation performed on the operation unit 10. Here, an operation of closing the clamp unit V3 is also performed.

In this step, if the substitution fluid is to be supplied through the pre-substitution line L2c switched from the physiological-saline supply line La, the control unit 11 activates the first substitution pump P3 and the substitution-fluid transfer pump P6 with the blood pump P1 stopped, and activates the second substitution pump P8. On the other hand, if the substitution fluid is to be supplied through the post-substitution line L2d switched from the physiological-saline supply line La (the pre-substitution line L2c), the control unit 11 activates the first substitution pump P3 and the substitution-fluid transfer pump P6 with the blood pump P1 stopped, but keeps the second substitution pump P8 stopped.

After the substitution pumps are activated in S6 (after the first substitution pump P3 and the substitution-fluid transfer pump P6 are activated and the second substitution pump P8 is selectively activated), it is checked in S7 whether a specified amount of returned blood is reached. The substitution pumps are kept in operation (the first substitution pump P3 and the substitution-fluid transfer pump P6 are kept in operation and the second substitution pump P8 is selectively kept in operation) until the specified amount of returned blood is reached. If it is determined that the specified amount is reached, blood return is ended. In this process of blood return, the identification of any clogging position by the clogging-identifying unit 7 can be performed immediately after the start of blood return.

Now, a blood purification apparatus according to a second embodiment of the present invention will be described.

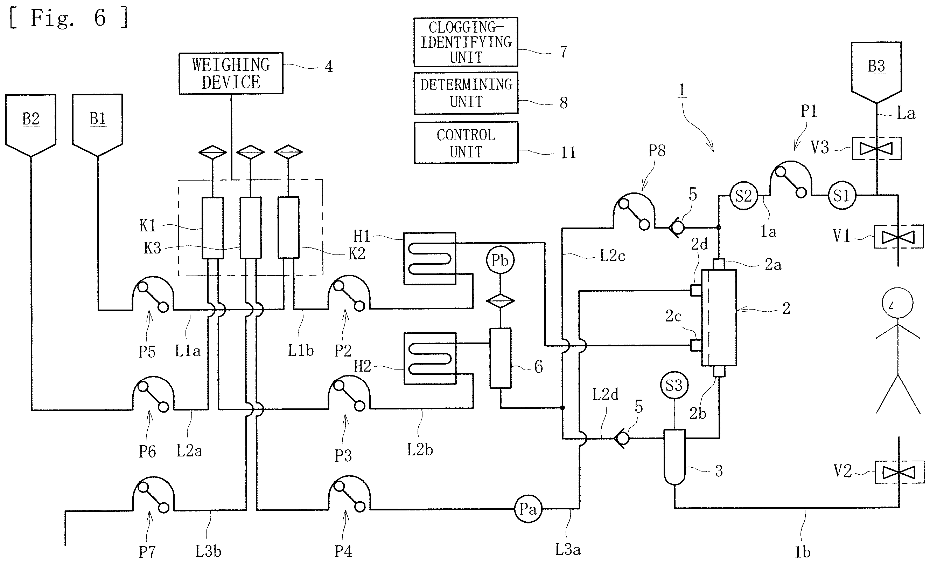

The blood purification apparatus according to the second embodiment is applied to a hemodialysis apparatus (particularly, a dialysis apparatus intended for continuous renal replacement therapy) for purifying blood of a patient while causing the blood to extracorporeally circulate. As illustrated in Fig. 6, the apparatus includes a blood circuit 1; a dialyzer 2 (blood purifier); a first dialysate introduction line L1a and a second dialysate introduction line L1b; a first substitution line L2a and a second substitution line L2b; a physiological-saline supply line La, which serves as a removal-side substitution-fluid supply line; a pre-substitution line L2c and a post-substitution line L2d, which each serve as a return-side substitution-fluid supply line; a first drain-liquid discharge line L3a and a second drain-liquid discharge line L3b; a blood pump P1; a dialysate introduction pump P2; a first substitution pump P3; a drain-liquid discharge pump P4; a dialysate transfer pump P5; a substitution-fluid transfer pump P6; a drain-liquid transfer pump P7; a second substitution pump P8; a reinfusion-fluid temporary chamber K1, a dialysate temporary chamber K2, and a drain-liquid temporary chamber K3; a weighing device 4; a clogging-identifying unit 7; a determining unit 8; and a control unit 11. Elements that are the same as those described in the first embodiment are denoted by corresponding ones of the reference signs, and detailed description of such elements is omitted.

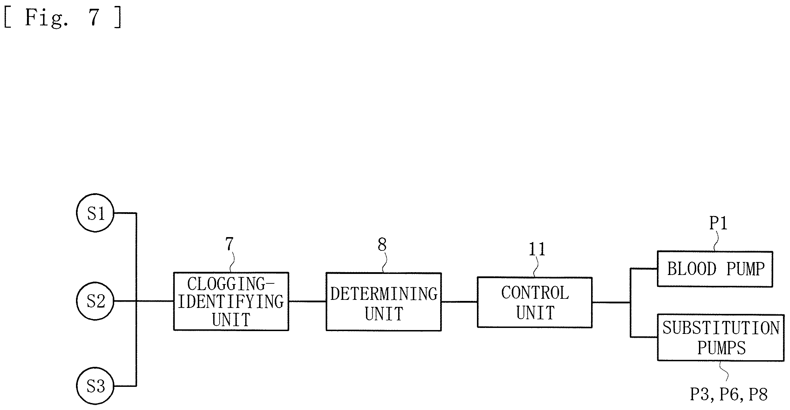

Referring to Fig. 7, the control unit 11 is a microcomputer or the like electrically connected to the determining unit 8, the blood pump P1, and the substitution pumps (the first substitution pump P3, the substitution-fluid transfer pump P6, and the second substitution pump P8). In the present embodiment, if it is determined by the determining unit 8 to switch the substitution-fluid supply line from one of the removal-side substitution-fluid supply line and the return-side substitution-fluid supply line to the other, the control unit 11 automatically switches the substitution-fluid supply line from the one of the removal-side substitution-fluid supply line and the return-side substitution-fluid supply line to the other. For example, at the time of blood return, if it is determined by the determining unit 8 to switch from the physiological-saline supply line La to the pre-substitution line L2c to supply the substitution fluid, the supply of the substitution fluid (physiological saline) through the physiological-saline supply line La is automatically switched to the supply of the substitution fluid (reinfusion fluid) through the pre-substitution line L2c. On the other hand, if it is determined by the determining unit 8 to switch from the physiological-saline supply line La or the pre-substitution line L2c to the post-substitution line L2d to supply the substitution fluid, the supply of the substitution fluid through the physiological-saline supply line La or the pre-substitution line L2c is automatically switched to the supply of the substitution fluid through the post-substitution line L2d.

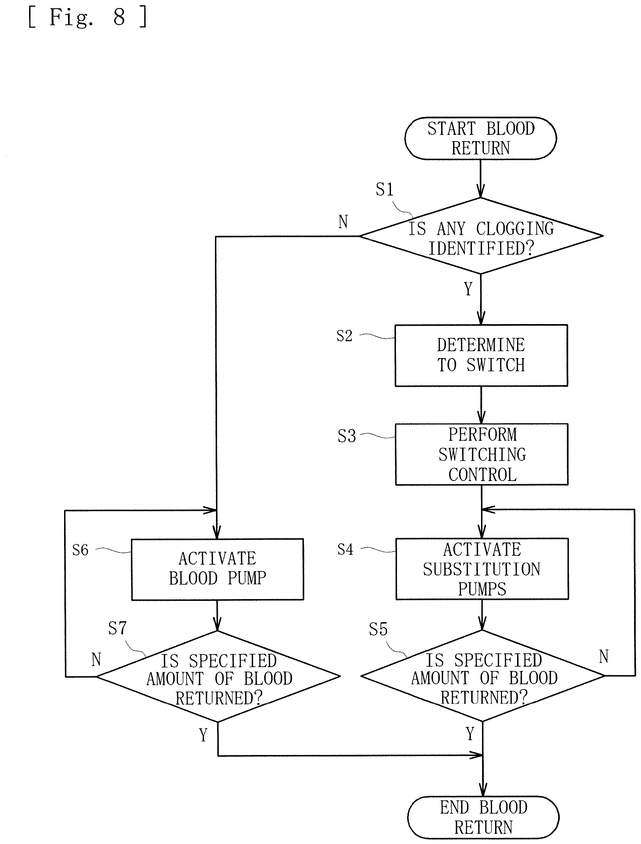

Now, a process of controlling blood return in the blood purification apparatus according to the second embodiment will be described with reference to a flow chart illustrated in Fig. 8.

After the completion of a blood purification treatment, blood return is started. Accordingly, the supply of the dialysate to the dialyzer 2 is stopped, and the blood pump P1 is stopped with the clamp unit V3 open. Thus, the physiological saline in the physiological-saline bag B3 is supplied to the blood circuit 1. With the self-weight of the physiological saline generated by the head difference, the blood in the flow route between the distal end E1 of the removal-side blood circuit 1a and the connection position E2 is substituted for by the substitution fluid, and the blood thus substituted for is returned to the patient.

Subsequently, the clamp unit V1 is closed, and the following process is performed to return the blood in the blood circuit 1 from the connection position E2 to the distal end E6 of the return-side blood circuit 1b. Specifically, in S1, it is checked whether any clogging position is identified by the clogging-identifying unit 7. If it is determined that no clogging position is identified (neither clogging nor narrowing is detected), the blood pump P1 is activated in S6. Then, in S7, it is checked whether a specified amount of returned blood is reached. In this step, the first substitution pump P3, the substitution-fluid transfer pump P6, and the second substitution pump P8 are kept stopped. Furthermore, the blood pump P1 is kept in operation until the specified amount of returned blood is reached. If it is determined that the specified amount is reached, blood return is ended.

On the other hand, if it is determined in S1 that a clogging position is identified, the determining unit 8 determines in S2 to switch the substitution-fluid supply lines. For example, if it is identified by the clogging-identifying unit 7 that clogging or narrowing is occurring between the connection position E2 of the physiological-saline supply line La and the installation position E3 of the blood pump P1, the determining unit 8 determines to switch from the physiological-saline supply line La to the pre-substitution line L2c to supply the substitution fluid. On the other hand, if it is identified by the clogging-identifying unit 7 that clogging or narrowing is occurring between the connection position E4 of the pre-substitution line L2c and the dialyzer 2, the determining unit 8 determines to switch from the physiological-saline supply line La or the pre-substitution line L2c to the post-substitution line L2d to supply the substitution fluid.

Subsequently, in S3, the control unit 11 performs switching control in accordance with the determination made by the determining unit 8. Furthermore, in S4, the control unit 11 activates the first substitution pump P3 and the substitution-fluid transfer pump P6 with the blood pump P1 kept stopped. In this step, if the substitution fluid is to be supplied through the pre-substitution line L2c, the control unit 11 activates the first substitution pump P3 and the substitution-fluid transfer pump P6 with the blood pump P1 stopped, and activates the second substitution pump P8. On the other hand, if the substitution fluid is to be supplied through the post-substitution line L2d, the control unit 11 activates the first substitution pump P3 and the substitution-fluid transfer pump P6 with the blood pump P1 stopped, but keeps the second substitution pump P8 stopped. Switching control described herein refers to, but is not limited to, a control operation of switching the states of operations of relevant elements including the blood pump P1, the first substitution pump P3, the substitution-fluid transfer pump P6, and the second substitution pump P8 (the states of operations of relevant actuators). Alternatively, switching control may be an operation of switching modes representing the substitution-fluid supply lines (the internal states of the blood purification apparatus). Furthermore, the operation of closing the clamp unit V3 may be performed in switching control.

After the substitution pumps are activated in S4 (after the first substitution pump P3 and the substitution-fluid transfer pump P6 are activated and the second substitution pump P8 is selectively activated), it is checked in S5 whether a specified amount of returned blood is reached. The substitution pumps are kept in operation (the first substitution pump P3 and the substitution-fluid transfer pump P6 are kept in operation and the second substitution pump P8 is selectively kept in operation) until the specified amount of returned blood is reached. If it is determined that the specified amount is reached, blood return is ended. In this process of blood return, the identification of any clogging position by the clogging-identifying unit 7 can be performed at the last phase of the blood purification treatment.

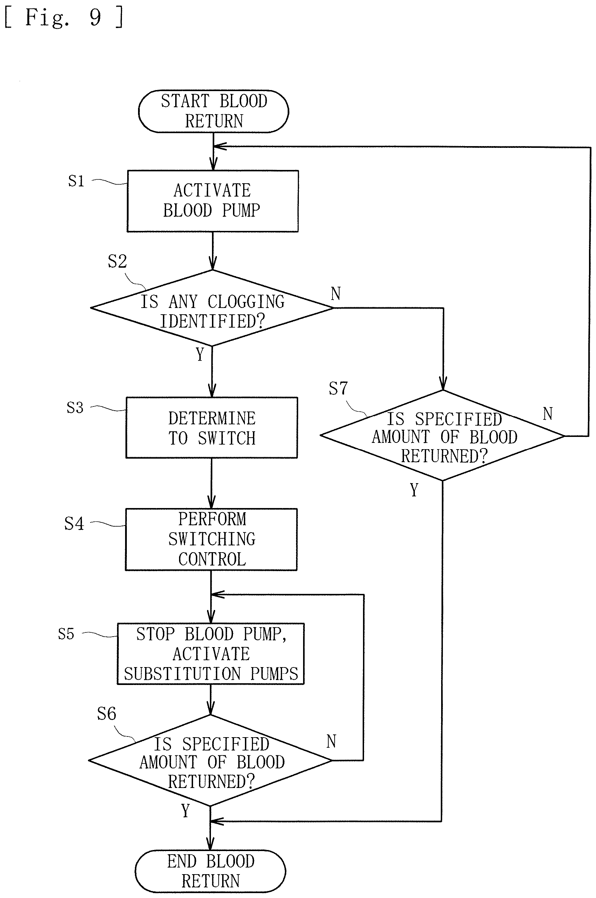

Now, another process of controlling blood return in the blood purification apparatus according to the second embodiment will be described with reference to a flow chart illustrated in Fig. 9.

After the completion of a blood purification treatment, blood return is started. Accordingly, the supply of the dialysate to the dialyzer 2 is stopped, and the blood pump P1 is stopped with the clamp unit V3 open. Thus, the physiological saline in the physiological-saline bag B3 is supplied to the blood circuit 1. With the self-weight of the physiological saline generated by the head difference, the blood in the flow route between the distal end E1 of the removal-side blood circuit 1a and the connection position E2 is substituted for by the substitution fluid, and the blood thus substituted for is returned to the patient.

Subsequently, the clamp unit V1 is closed, and the following process is performed to return the blood in the blood circuit 1 from the connection position E2 to the distal end E6 of the return-side blood circuit 1b. Specifically, in S1, the blood pump P1 is activated. Then, in S2, it is checked whether any clogging position is identified by the clogging-identifying unit 7. If it is determined that no clogging position is identified (neither clogging nor narrowing is detected), it is checked in S7 whether a specified amount of returned blood is reached. In this step, the first substitution pump P3, the substitution-fluid transfer pump P6, and the second substitution pump P8 are kept stopped. Furthermore, the blood pump P1 is kept in operation until the specified amount of returned blood is reached. If it is determined that the specified amount is reached, blood return is ended.

On the other hand, if it is determined in S2 that a clogging position is identified, the determining unit 8 determines in S3 to switch the substitution-fluid supply lines. For example, if it is identified by the clogging-identifying unit 7 that clogging or narrowing is occurring between the connection position E2 of the physiological-saline supply line La and the installation position E3 of the blood pump P1, the determining unit 8 determines to switch from the physiological-saline supply line La to the pre-substitution line L2c to supply the substitution fluid. On the other hand, if it is identified by the clogging-identifying unit 7 that clogging or narrowing is occurring between the connection position E4 of the pre-substitution line L2c and the dialyzer 2, the determining unit 8 determines to switch from the physiological-saline supply line La or the pre-substitution line L2c to the post-substitution line L2d to supply the substitution fluid.

Subsequently, in S4, the control unit 11 performs switching control in accordance with the determination made by the determining unit 8. Furthermore, in S5, the control unit 11 stops the blood pump P1 but activates the first substitution pump P3 and the substitution-fluid transfer pump P6. In this step, if the substitution fluid is to be supplied through the pre-substitution line L2c switched from the physiological-saline supply line La, the control unit 11 activates the first substitution pump P3 and the substitution-fluid transfer pump P6 with the blood pump P1 stopped, and activates the second substitution pump P8. On the other hand, if the substitution fluid is to be supplied through the post-substitution line L2d switched from the physiological-saline supply line La, the control unit 11 activates the first substitution pump P3 and the substitution-fluid transfer pump P6 with the blood pump P1 stopped, but keeps the second substitution pump P8 stopped. Switching control described herein refers to, but is not limited to, a control operation of switching the states of operations of relevant elements including the blood pump P1, the first substitution pump P3, the substitution-fluid transfer pump P6, and the second substitution pump P8 (the states of operations of relevant actuators). Alternatively, switching control may be an operation of switching modes representing the substitution-fluid supply lines (the internal states of the blood purification apparatus). Furthermore, the operation of closing the clamp unit V3 may be performed in switching control.

After the substitution pumps are activated in S5 (after the first substitution pump P3 and the substitution-fluid transfer pump P6 are activated and the second substitution pump P8 is selectively activated), it is checked in S6 whether a specified amount of returned blood is reached. The substitution pumps are kept in operation (the first substitution pump P3 and the substitution-fluid transfer pump P6 are kept in operation and the second substitution pump P8 is selectively kept in operation) until the specified amount of returned blood is reached. If it is determined that the specified amount is reached, blood return is ended. In this process of blood return, the identification of any clogging position by the clogging-identifying unit 7 can be performed immediately after the start of blood return.

Now, a blood purification apparatus according to a third embodiment of the present invention will be described.

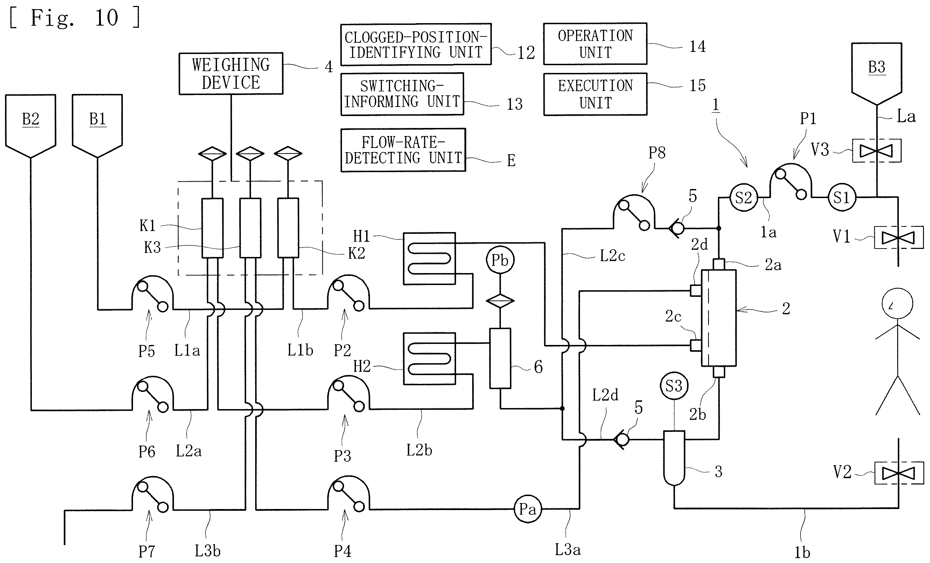

The blood purification apparatus according to the third embodiment is applied to a hemodialysis apparatus (particularly, a dialysis apparatus intended for continuous renal replacement therapy) for purifying blood of a patient while causing the blood to extracorporeally circulate. As illustrated in Fig. 10, the apparatus includes a blood circuit 1; a dialyzer 2 (blood purifier); a first dialysate introduction line L1a and a second dialysate introduction line L1b; a first substitution line L2a and a second substitution line L2b; a physiological-saline supply line La, which serves as a removal-side substitution-fluid supply line; a pre-substitution line L2c and a post-substitution line L2d, which each serve as a return-side substitution-fluid supply line; a first drain-liquid discharge line L3a and a second drain-liquid discharge line L3b; a blood pump P1; a dialysate introduction pump P2; a first substitution pump P3; a drain-liquid discharge pump P4; a dialysate transfer pump P5; a substitution-fluid transfer pump P6; a drain-liquid transfer pump P7; a second substitution pump P8; a reinfusion-fluid temporary chamber K1, a dialysate temporary chamber K2, and a drain-liquid temporary chamber K3; a weighing device 4; a flow-rate-detecting unit E; a clogging-position-identifying unit 12; a switching-informing unit 13; an operation unit 14; and an execution unit 15. Elements that are the same as those described in the first embodiment are denoted by corresponding ones of the reference signs, and detailed description of such elements is omitted.

The flow-rate-detecting unit E is configured to detect the flow rate at a specific position of the flow route in the blood circuit 1 and is capable of detecting the flow rate of, for example, the priming solution during priming or the blood during blood purification treatment. The flow-rate-detecting unit E may be based on any of various detection technologies including the following, for example: a device configured to detect the flow rate with reference to the driving speed of the pump such as the blood pump P1, a device configured to directly detect the flow rate of the fluid in the blood circuit 1, and the like.

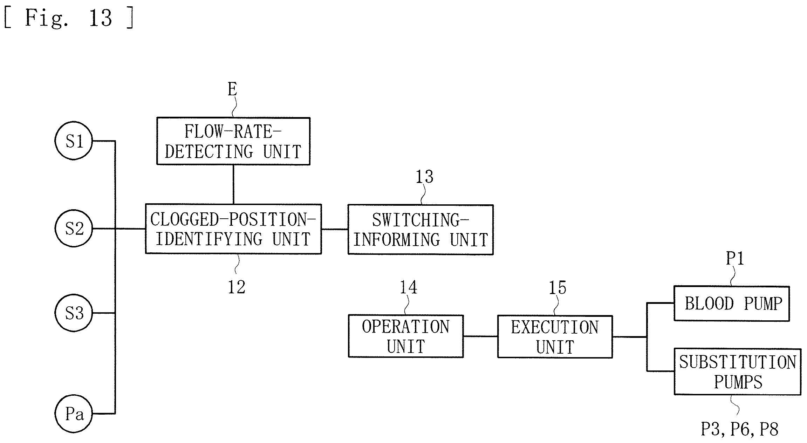

The clogging-position-identifying unit 12 is, for example, a microcomputer, a memory, or the like. As illustrated in Fig. 13, the clogging-position-identifying unit 12 is electrically connected to each of the pressure sensors (S1 to S3) and the flow-rate-detecting unit E and is capable of receiving the pressures detected by the pressure sensors (S1 to S3) and the flow rate detected by the flow-rate-detecting unit E. The clogging-position-identifying unit 12 according to the present embodiment is connected not only to the pressure sensors (S1 to S3) provided to the blood circuit 1 but also to the pressure sensor Pa provided to the first drain-liquid discharge line L3a, and is capable of receiving the pressure at the pressure sensor Pa. Alternatively, the clogging-position-identifying unit 12 may be connected exclusively to the pressure sensors (S1 to S3).

The clogging-position-identifying unit 12 according to the present embodiment uses a learned model obtained through machine learning in which a plurality of pieces of data on pressure changes and flow rates at the occurrences of clogging or narrowing of the flow route in the blood circuit 1 are accumulated to generate cumulative data to be used as teaching data for identifying any clogging position of the flow route. Thus, the clogging-position-identifying unit 12 identifies any clogging position with reference to the pressure changes and the flow rate detected by the pressure sensors (S1 to S3) and the flow-rate-detecting unit E. Machine learning refers to a technology of automatically building algorithms or models for achieving various tasks, such as classification and prediction, by using a computer learning a large amount of data. Technologies and algorithms for implementing machine learning include neural networks, for example.

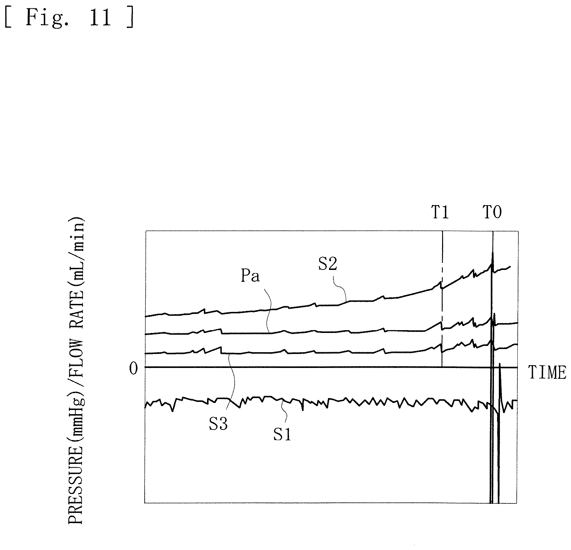

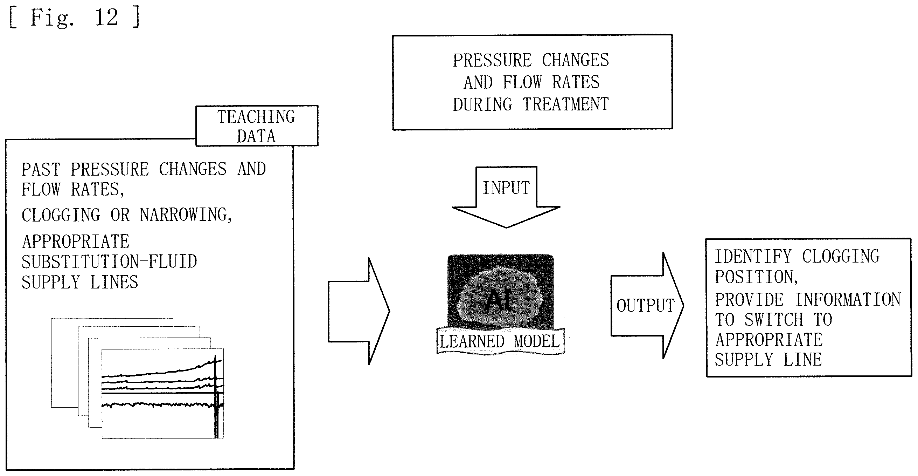

Specific content of machine learning according to the present embodiment will now be described with reference to Figs. 11 and 12.

A plurality of pieces of (a large amount of) data on pressure changes and flow rates at the occurrences of clogging or narrowing of the flow route in the blood circuit are accumulated in advance in a storage device such as a server, whereby cumulative data is generated. For example, if the flow route is narrowed at the installation position of the pressure sensor S2, as illustrated in Fig. 11, the pressure and the flow rate increase with time, starting from time T1. Then, if the flow route is clogged at time T0, the pressure sharply drops, reducing the flow rate to zero. Such changes in the pressure and the flow rate in the flow route are characterized in being greater than in a case where the flow route is neither clogged nor narrowed.

Such data on pressure changes and flow rates at the occurrences of clogging or narrowing of the flow route in the blood circuit is accumulated in a plural number (by a large amount) in a storage device such as a server, is then inputted as teaching data to a computer for machine learning, and is put to machine learning so that a specific position where clogging or narrowing has occurred (a clogging position) can be identified. The clogging-position-identifying unit 12 stores a learned model (such as a program) obtained through the above machine learning and uses the learned model to identify any clogging position at time T0 with reference to the pressure changes and the flow rate detected by the pressure sensors (S1 to S3) and the flow-rate-detecting unit E.

Furthermore, the clogging-position-identifying unit 12 according to the present embodiment uses a learned model obtained through machine learning in which not only a plurality of pieces of data on pressure changes and flow rates at the occurrences of clogging or narrowing of the flow route in the blood circuit 1 but also a plurality of pieces of data representing switching to an appropriate one of the substitution-fluid supply lines at the identification of a clogging position are accumulated to generate cumulative data to be used as teaching data for identifying any clogging position and for switching to an appropriate one of the substitution-fluid supply lines at the identification of a clogging position. Thus, the clogging-position-identifying unit 12 identifies any clogging position with reference to the pressures and the flow rate detected by the pressure sensors (S1 to S3) and the flow-rate-detecting unit E, and determines to switch to an appropriate one of the substitution-fluid supply lines at the identification of a clogging position.

The switching-informing unit 13 is an output device electrically connected to the clogging-position-identifying unit 12. If a clogging position is identified and it is determined by the clogging-position-identifying unit 12 to switch the substitution-fluid supply lines, the switching-informing unit 13 provides information to switch the substitution-fluid supply lines at the time of blood return. The switching-informing unit 13 is any of the following, for example: a display device, such as a monitor, configured to display an image that prompts switching; an output device, such as a speaker, configured to output a sound that prompts switching; an illumination device, such as an LED, configured to emit light or blink in such a manner as to prompt switching; and the like.

For example, at the time of blood return, if it is determined by the clogging-position-identifying unit 12 to switch from the physiological-saline supply line La to the pre-substitution line L2c to supply the substitution fluid, the switching-informing unit 13 provides information that prompts an operation of supplying the substitution fluid (reinfusion fluid) through the pre-substitution line L2c instead of supplying the substitution fluid (physiological saline) through the physiological-saline supply line La. On the other hand, if it is determined by the clogging-position-identifying unit 12 to switch from the physiological-saline supply line La or the pre-substitution line L2c to the post-substitution line L2d to supply the substitution fluid, the switching-informing unit 13 provides information that prompts an operation of supplying the substitution fluid through the post-substitution line L2d instead of supplying the substitution fluid through the physiological-saline supply line La or the pre-substitution line L2c.

At the time of blood return, if the information prompting to switch the substitution-fluid supply lines is provided by the switching-informing unit 13, an operation for the switching is to be performed on the operation unit 14. For example, an operation of opening or closing the clamp unit V1 and an operation of activating or stopping relevant pumps can be performed on the operation unit 14. More specifically, the supply of the substitution fluid (physiological saline) through the physiological-saline supply line La may be switched to the supply of the substitution fluid (reinfusion fluid) through the pre-substitution line L2c, by stopping the operation of the blood pump P1 with the clamp unit V1 closed and activating the second substitution pump P8 with the first substitution pump P3 and the substitution-fluid transfer pump P6 being in operation.

On the other hand, the supply of the substitution fluid (reinfusion fluid) through the pre-substitution line L2c may be switched to the supply of the substitution fluid (reinfusion fluid) through the post-substitution line L2d, by keeping the clamp unit V1 closed and the blood pump P1 stopped and stopping the second substitution pump P8 with the first substitution pump P3 and the substitution-fluid transfer pump P6 being in operation. Furthermore, the supply of the substitution fluid (physiological saline) through the physiological-saline supply line La may be switched to the supply of the substitution fluid (reinfusion fluid) through the post-substitution line L2d, by stopping the operation of the blood pump P1 with the clamp unit V1 closed and stopping the second substitution pump P8 with the first substitution pump P3 and the substitution-fluid transfer pump P6 being in operation.

The execution unit 15 is configured to switch the substitution-fluid supply line from one of the removal-side substitution-fluid supply line and the return-side substitution-fluid supply line to the other. Specifically, referring to Fig. 13, the execution unit 15 according to the present embodiment is a microcomputer or the like electrically connected to each of the operation unit 14, the blood pump P1, the first substitution pump P3, the substitution-fluid transfer pump P6, and the second substitution pump P8. If a predetermined operation is performed by the operator in response to the information provided by the switching-informing unit 13, the execution unit 15 switches the substitution-fluid supply line from one of the removal-side substitution-fluid supply line (physiological-saline supply line) and the return-side substitution-fluid supply line (pre-substitution line L2c or post-substitution line L2d) to the other.

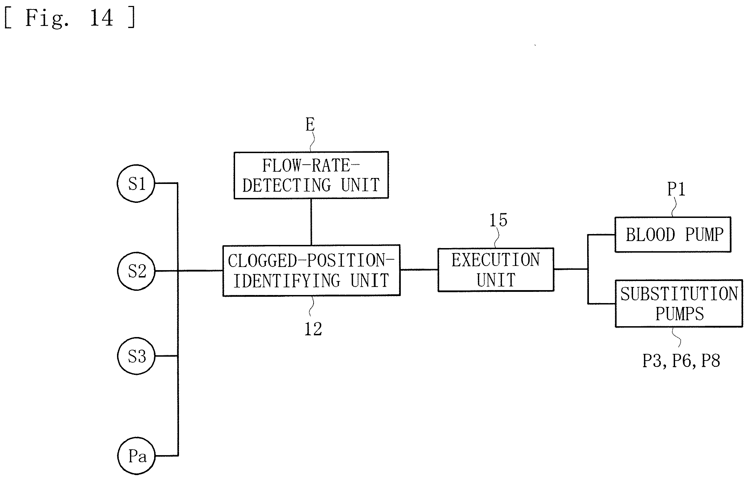

Alternatively, referring to Fig. 14, the execution unit 15 may be a microcomputer or the like electrically connected to the clogging-position-identifying unit 12, the blood pump P1, and the substitution pumps (the first substitution pump P3, the substitution-fluid transfer pump P6, and the second substitution pump P8) and may be configured not only to switch the substitution-fluid supply line from one of the removal-side substitution-fluid supply line and the return-side substitution-fluid supply line to the other, but also to, if a clogging position is identified by the clogging-position-identifying unit 12 and it is determined to switch to an appropriate one of the substitution-fluid supply lines, automatically switch to the one of the substitution-fluid supply lines that is determined to be appropriate. In that case, for example, at the time of blood return, if it is determined by the clogging-position-identifying unit 12 to switch from the physiological-saline supply line La to the pre-substitution line L2c to supply the substitution fluid, the supply of the substitution fluid (physiological saline) through the physiological-saline supply line La is automatically switched to the supply of the substitution fluid (reinfusion fluid) through the pre-substitution line L2c. On the other hand, if it is determined by the clogging-position-identifying unit 12 to switch from the physiological-saline supply line La or the pre-substitution line L2c to the post-substitution line L2d to supply the substitution fluid, the supply of the substitution fluid through the physiological-saline supply line La or the pre-substitution line L2c is automatically switched to the supply of the substitution fluid through the post-substitution line L2d.

Now, a blood purification apparatus according to a fourth embodiment of the present invention will be described.

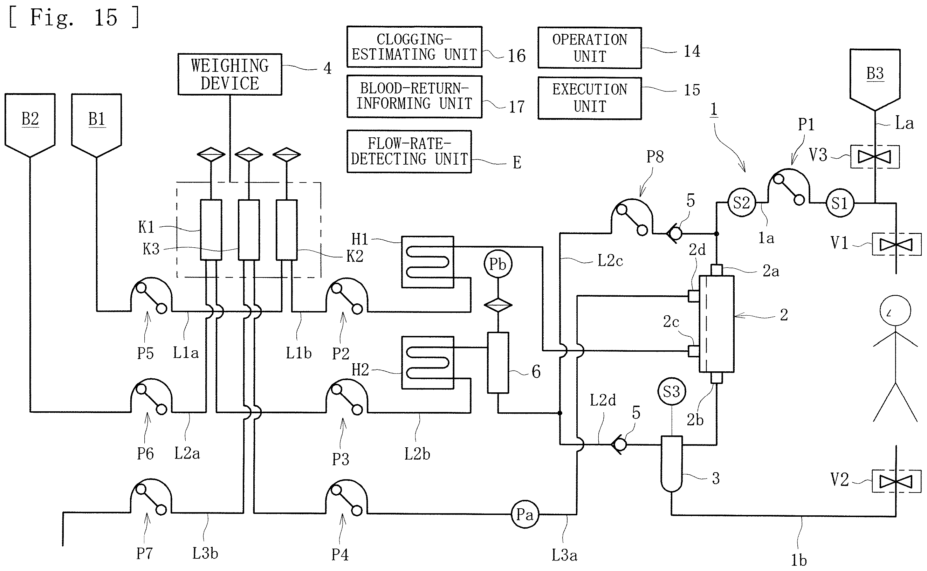

The blood purification apparatus according to the fourth embodiment is applied to a hemodialysis apparatus (particularly, a dialysis apparatus intended for continuous renal replacement therapy) for purifying blood of a patient while causing the blood to extracorporeally circulate. As illustrated in Fig. 15, the apparatus includes a blood circuit 1; a dialyzer 2 (blood purifier); a first dialysate introduction line L1a and a second dialysate introduction line L1b; a first substitution line L2a and a second substitution line L2b; a physiological-saline supply line La, which serves as a removal-side substitution-fluid supply line; a pre-substitution line L2c and a post-substitution line L2d, which each serve as a return-side substitution-fluid supply line; a first drain-liquid discharge line L3a and a second drain-liquid discharge line L3b; a blood pump P1; a dialysate introduction pump P2; a first substitution pump P3; a drain-liquid discharge pump P4; a dialysate transfer pump P5; a substitution-fluid transfer pump P6; a drain-liquid transfer pump P7; a second substitution pump P8; a reinfusion-fluid temporary chamber K1, a dialysate temporary chamber K2, and a drain-liquid temporary chamber K3; a weighing device 4; a flow-rate-detecting unit E; a clogging-estimating unit 16; a blood-return-informing unit 17; an operation unit 14; and an execution unit 15. Elements that are the same as those described in the first or third embodiment are denoted by corresponding ones of the reference signs, and detailed description of such elements is omitted.

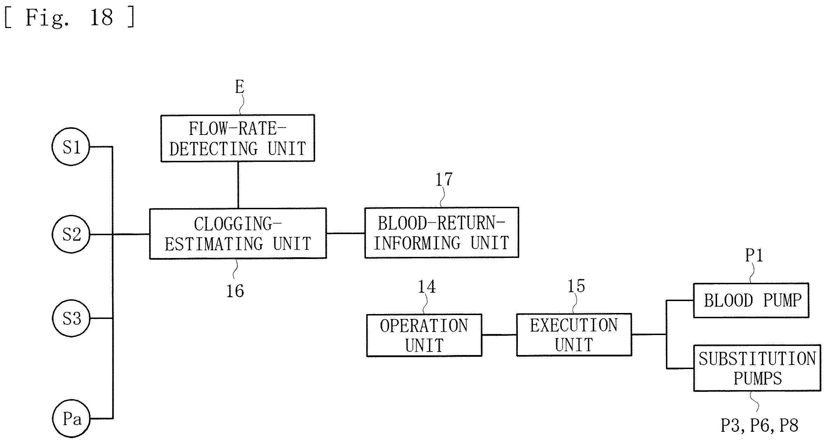

The clogging-estimating unit 16 is, for example, a microcomputer, a memory, or the like. As illustrated in Fig. 18, the clogging-estimating unit 16 is electrically connected to each of the pressure sensors (S1 to S3) and the flow-rate-detecting unit E and is capable of receiving the pressures detected by the pressure sensors (S1 to S3) and the flow rate detected by the flow-rate-detecting unit E. The clogging-estimating unit 16 according to the present embodiment is connected not only to the pressure sensors (S1 to S3) provided to the blood circuit 1 but also to the pressure sensor Pa provided to the first drain-liquid discharge line L3a, and is capable of receiving the pressure at the pressure sensor Pa. Alternatively, the clogging-estimating unit 16 may be connected exclusively to the pressure sensors (S1 to S3).

The clogging-estimating unit 16 according to the present embodiment uses a learned model obtained through machine learning in which a plurality of pieces of data on pressure changes and flow rates at the occurrences of clogging or narrowing of the flow route in the blood circuit 1 are accumulated to generate cumulative data to be used as teaching data for identifying any clogging position of the flow route. Thus, the clogging-estimating unit 16 estimates any clogging position that is to occur in a predetermined time period, with reference to the pressure changes and the flow rate detected by the pressure sensors (S1 to S3) and the flow-rate-detecting unit E. For machine learning, refer to the description provided for the third embodiment.

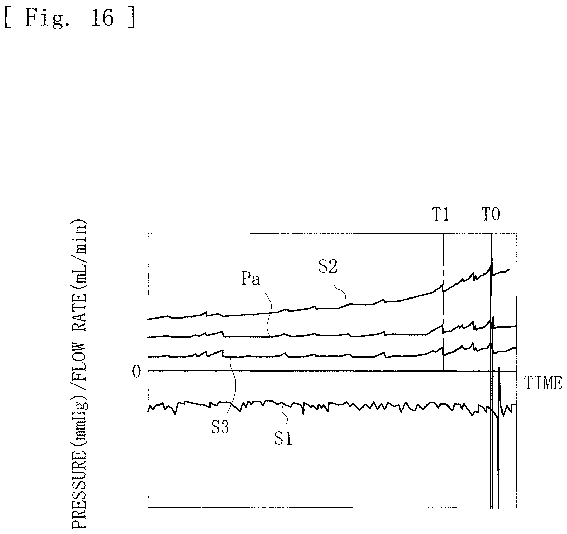

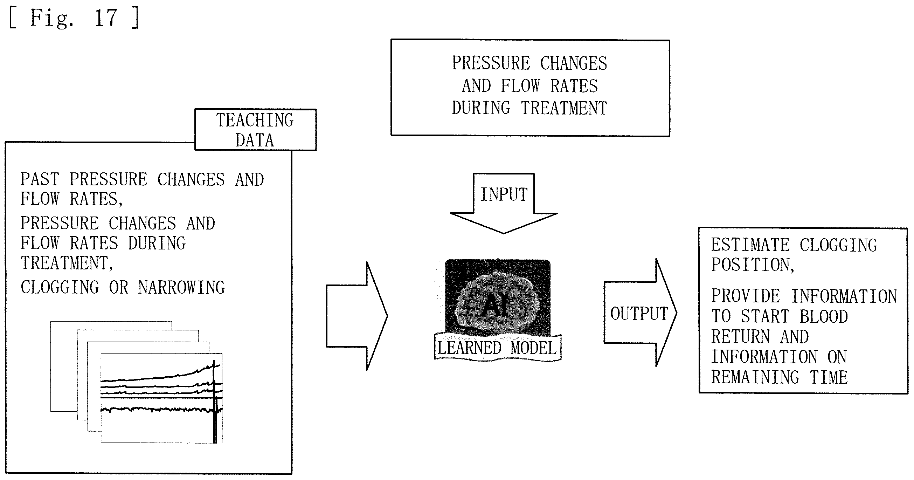

Specific content of machine learning according to the present embodiment will now be described with reference to Figs. 16 and 17.

A plurality of pieces of (a large amount of) data on pressure changes and flow rates at the occurrences of clogging or narrowing of the flow route in the blood circuit are accumulated in advance in a storage device such as a server, whereby cumulative data is generated. For example, if the flow route is narrowed at the installation position of the pressure sensor S2, as illustrated in Fig. 16, the pressure and the flow rate increase with time, starting from time T1. Then, if the flow route is clogged at time T0, the pressure sharply drops, reducing the flow rate to zero. Such changes in the pressure and the flow rate in the flow route are characterized in being greater than in a case where the flow route is neither clogged nor narrowed.

Such data on pressure changes and flow rates at the occurrences of clogging or narrowing of the flow route in the blood circuit is accumulated in a plural number (by a large amount) in a storage device such as a server, is then inputted as teaching data to a computer for machine learning, and is put to machine learning so that a specific position where clogging or narrowing has occurred (a clogging position) can be identified. The clogging-estimating unit 16 stores a learned model (such as a program) obtained through the above machine learning and uses the learned model to estimate any clogging position that is to occur in a predetermined time period from time T1 (that is, to estimate any clogging position that is to occur at time T0 if the blood purification treatment is continued as is), with reference to the pressure changes and the flow rate detected by the pressure sensors (S1 to S3) and the flow-rate-detecting unit E.

Furthermore, the clogging-estimating unit 16 according to the present embodiment uses teaching data generated from pressure changes and flow rates detected by the pressure sensors (S1 to S3) and the flow-rate-detecting unit E in the process of priming or blood purification treatment, learns whether the pressure changes and the flow rates detected by the pressure sensors (S1 to S3) and the flow-rate-detecting unit E are within predetermined ranges, and estimates any clogging position that is to occur in a predetermined time period (at time T0) with reference to changes in the pressures and the flow rate detected until the current time, T1, by the pressure sensors (S1 to S3) and the flow-rate-detecting unit E.

The blood-return-informing unit 17 is an output device electrically connected to the clogging-estimating unit 16. If a clogging position is estimated to occur in a predetermined time period by the clogging-estimating unit 16, the blood-return-informing unit 17 provides information to start blood return. The blood-return-informing unit 17 is any of the following, for example: a display device, such as a monitor, configured to display an image that prompts to start blood return; an output device, such as a speaker, configured to output a sound that prompts to start blood return; an illumination device, such as an LED, configured to emit light or blink in such a manner as to prompt to start blood return; and the like.

For example, if a clogging position is estimated to occur in a predetermined time period, or at time T0, by the clogging-estimating unit 16, the blood-return-informing unit 17 can provide information to end the current blood purification treatment and to perform blood return, and can further provide information on the remaining time before the flow route is estimated to be clogged or narrowed (the period from current time T1 to time T0). The information to perform blood return may be provided as information on how to control the operation (the flow rate at the time of blood return) of any of the pumps, including the blood pump P1.

If the information to start blood return is provided by the blood-return-informing unit 17, an operation for blood return is to be performed on the operation unit 14. For example, an operation of opening or closing the clamp unit V1 and an operation of activating or stopping relevant pumps can be performed on the operation unit 14. For example, if the information to start blood return is provided by the blood-return-informing unit 17, the blood pump P1 may be activated with the clamp unit V3 open but the clamp unit V1 closed. Thus, the substitution fluid (physiological saline) is supplied through the physiological-saline supply line La to substitute for the blood in the blood circuit 1, and blood return is achieved.

The execution unit 15 is configured to start blood return. Referring to Fig. 18, the execution unit 15 is a microcomputer or the like electrically connected to each of the operation unit 14, the blood pump P1, the first substitution pump P3, the substitution-fluid transfer pump P6, and the second substitution pump P8. If a predetermined operation is performed by the operator in response to the information provided by the blood-return-informing unit 17, the execution unit 15 opens the substitution-fluid supply line to start blood return.

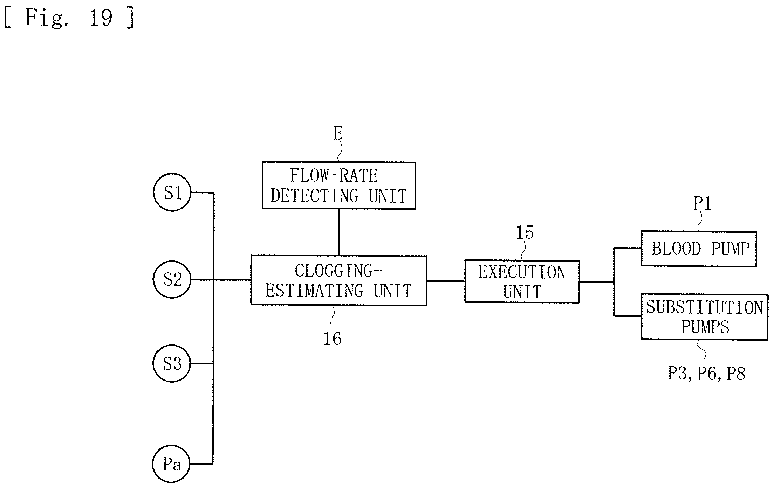

Alternatively, referring to Fig. 19, the execution unit 15 may be a microcomputer or the like electrically connected to the clogging-estimating unit 16, the blood pump P1, and the substitution pumps (the first substitution pump P3, the substitution-fluid transfer pump P6, and the second substitution pump P8) and may be configured to automatically switch to blood return if a clogging position is estimated to occur in a predetermined time period by the clogging-estimating unit 16. In that case, if it is estimated by the clogging-estimating unit 16 that clogging or narrowing of the flow route may occur in a predetermined time period, priming or blood purification treatment is automatically ended. Then, blood return is automatically started with the supply of the substitution fluid.

According to the first to third embodiments described above, the substitution-fluid supply line is to be switched from one of the removal-side substitution-fluid supply line (physiological-saline supply line La) and the return-side substitution-fluid supply line (pre-substitution line L2c or post-substitution line L2d) to the other. Therefore, even if clogging or narrowing occurs in the blood flow route at the time of blood return, the blood remaining in the blood circuit 1 can be smoothly returned into the patient's body, with no need of reconnection of the substitution-fluid supply line during blood return.

In particular, the first embodiment employs the informing unit 9 configured to provide information to switch the substitution-fluid supply lines if it is determined by the determining unit 8 to switch the substitution-fluid supply line from one of the removal-side substitution-fluid supply line (physiological-saline supply line La) and the return-side substitution-fluid supply line (pre-substitution line L2c or post-substitution line L2d) to the other. Therefore, the operator can be prompted to switch the substitution-fluid supply lines in accordance with the determination made by the determining unit 8.

The second embodiment employs the control unit 11 configured to automatically switch the substitution-fluid supply lines if it is determined by the determining unit 8 to switch the substitution-fluid supply line from one of the removal-side substitution-fluid supply line (physiological-saline supply line La) and the return-side substitution-fluid supply line (pre-substitution line L2c or post-substitution line L2d) to the other. Therefore, switching of the substitution-fluid supply lines can be automatically performed in accordance with the determination made by the determining unit 8.

According to the first and second embodiments, if a clogging position is identified at an upstream position (a position between the distal end E1 of the removal-side blood circuit 1a and the installation position E3 of the blood pump P1) by the clogging-identifying unit 7, the determining unit 8 determines to switch from the removal-side substitution-fluid supply line (physiological-saline supply line La) to the return-side substitution-fluid supply line (pre-substitution line L2c or post-substitution line L2d) to supply the substitution fluid at the time of blood return. Therefore, blood return can be performed without reversely rotating the blood pump P1.

According to the first and second embodiments, the return-side substitution-fluid supply line includes a plurality of lines connected to a return-side part of the blood circuit 1. Furthermore, the blood purification apparatus includes the first pressure sensor S1 configured to detect the pressure at a removal-side position of the blood circuit 1, and the second pressure sensor S2 and the third pressure sensor S3 configured to detect the pressures on the removal side and the return side, respectively, of the dialyzer 2 at the respective return-side positions of the blood circuit 1. Any clogging position is identified with reference to the pressures detected by the first pressure sensor S1, the second pressure sensor S2, and the third pressure sensor S3, and the removal-side substitution-fluid supply line is switched to either of the return-side substitution-fluid supply lines (the pre-substitution line L2c or the post-substitution line L2d) to supply the substitution fluid. Therefore, accurate identification of any clogging position is achieved with reference to the pressures detected by the pressure units.

According to the first and second embodiments, the return-side substitution-fluid supply line includes the pre-substitution line L2c connected to the removal-side blood circuit 1a and through which the substitution fluid is allowed to be supplied, and the post-substitution line L2d connected to the return-side blood circuit 1b and through which the substitution fluid is allowed to be supplied. Furthermore, the determining unit 8 is configured to determine to switch from the removal-side substitution-fluid supply line (physiological-saline supply line La) to the pre-substitution line L2c or the post-substitution line L2d to supply the substitution fluid. Therefore, the pre-substitution line L2c or the post-substitution line L2d intended for performing pre-substitution or post-substitution during treatment can be utilized to supply the substitution fluid.

The first and second embodiments employ a peristaltic substitution pump (the second substitution pump P8) configured to supply the substitution fluid to the blood circuit 1 through the pre-substitution line L2c or the post-substitution line L2d. Therefore, the amount of the substitution fluid supplied at the time of blood return can be known accurately, and the end time of blood return can be known accurately.

According to the third embodiment, a learned model obtained through machine learning is used to identify any clogging position with reference to the pressure changes and the flow rate detected by the pressure sensors (S1 to S3) and the flow-rate-detecting unit E. Therefore, accurate identification of any clogging position is achieved by utilizing the learned model. According to the fourth embodiment, a learned model obtained through machine learning is used to estimate any clogging position that is to occur in a predetermined time period with reference to the pressure changes and the flow rate detected by the pressure sensors (S1 to S3) and the flow-rate-detecting unit E. Therefore, accurate estimation of possible clogging or narrowing is achieved by utilizing the learned model.

While some embodiments have been described above, the present invention is not limited thereto. The removal-side substitution-fluid supply line may be a supply line different from the physiological-saline supply line La. For example, the removal-side substitution-fluid supply line may be a supply line branching off from the second dialysate introduction line L1b and connected to the connection position E2 (in such a case, the substitution fluid for blood return is the dialysate), or a supply line branching off from the second substitution line L2b and connected to the connection position E2 (in such a case, the substitution fluid for blood return is the reinfusion fluid).

While the return-side substitution-fluid supply line according to the above embodiments consists of the pre-substitution line L2c and the post-substitution line L2d, the return-side substitution-fluid supply line may be a supply line different therefrom (but a supply line connected to a downstream position), and may consist of a single line or three or more lines. That is, the second dialysate introduction line L1b or the first drain-liquid discharge line L3a connected to the dialyzer 2 (blood purifier) may be utilized as the return-side substitution-fluid supply line. In that case, the dialysate serving as the substitution fluid is to be supplied by way of back-filtration from the dialysate flow routes to the blood flow routes in the dialyzer 2.

According to the above embodiments, if a clogging position is identified at an upstream position by the clogging-identifying unit 7, the determining unit 8 determines to switch from the removal-side substitution-fluid supply line to the return-side substitution-fluid supply line to supply the substitution fluid at the time of blood return. Alternatively, if a clogging position is identified at a downstream position by the clogging-identifying unit 7, the determining unit 8 may determine to switch from the return-side substitution-fluid supply line to the removal-side substitution-fluid supply line to supply the substitution fluid at the time of blood return.

While the above embodiments are applied to a dialysis apparatus intended for continuous renal replacement therapy (CRRT), the above embodiments are also applicable to other apparatuses such as a dialysis apparatus intended for patients suffering from chronic renal failure, and an apheresis apparatus configured to remove a specific component from blood. Furthermore, the substitution fluid to be used at the time of blood return is not limited to physiological saline, dialysate, and reinfusion fluid and may be another fluid (liquid containing electrolyte) that is suitable for substitution to be performed at the time of blood return.

### Industrial Applicability

The present invention is also applicable to any blood purification apparatus having a different appearance, additional functions, or the like within the spirit of the present invention.

### Reference Signs List

1 blood circuit
1a removal-side blood circuit
1b return-side blood circuit
2 dialyzer (blood purifier)
3 air-trap chamber
4 weighing device
5 heating bag
6 air-trap chamber
7 clogging-identifying unit
8 determining unit
9 informing unit
10 operation unit
11 control unit
12 clogging-position-identifying unit
13 switching-informing unit
14 operation unit
15 execution unit
16 clogging-estimating unit
17 blood-return-informing unit
K1 reinfusion-fluid temporary chamber
K2 dialysate temporary chamber
K3 drain-liquid temporary chamber
B1 dialysate bag
B2 reinfusion-fluid bag
B3 physiological-saline bag
L1a first dialysate introduction line
L1b second dialysate introduction line
L2a first substitution line
L2b second substitution line
L2c pre-substitution line (return-side substitution-fluid supply line)
L2d post-substitution line (return-side substitution-fluid supply line)
L3a first drain-liquid discharge line
L3b second drain-liquid discharge line
La physiological-saline supply line (removal-side substitution-fluid supply line)
P1 blood pump
P2 dialysate introduction pump
P3 first substitution pump
P4 drain-liquid discharge pump
P5 dialysate transfer pump
P6 substitution-fluid transfer pump
P7 drain-liquid transfer pump
P8 second substitution pump
H1 heating device
H2 heating device
S1 first pressure sensor (first detecting unit)
S2 second pressure sensor (second detecting unit)
S3 third pressure sensor (third detecting unit)
Pa, Pb pressure sensor
V1 to V3 clamp unit
E flow-rate-detecting unit

## Claims

1. A blood purification apparatus comprising:
a blood circuit including a removal-side blood circuit for removing blood from a patient and a return-side blood circuit for returning the blood to the patient, the blood of the patient being caused to extracorporeally circulate through the blood circuit with a blood purifier being connected to the removal-side blood circuit and to the return-side blood circuit, the blood purifier being configured to purify the blood;
a blood pump provided to the blood circuit and configured to cause the blood of the patient to extracorporeally circulate through the blood circuit;
a removal-side substitution-fluid supply line connected to the blood circuit and through which substitution fluid is allowed to be supplied to the blood circuit at a time of blood return;
a return-side substitution-fluid supply line connected to a position of the blood circuit or to the blood purifier and through which the substitution fluid is allowed to be supplied to the blood circuit at the time of blood return, the position of the blood circuit being on a return side relative to a part to which the removal-side substitution-fluid supply line is connected; and
a control unit configured to switch the substitution-fluid supply line from one of the removal-side substitution-fluid supply line and the return-side substitution-fluid supply line to an other.

2. The blood purification apparatus according to Claim 1, further comprising an informing unit configured to provide information to switch the substitution-fluid supply lines if it is determined to switch the substitution-fluid supply line from one of the removal-side substitution-fluid supply line and the return-side substitution-fluid supply line to an other, wherein if a predetermined operation is performed by an operator in response to the information provided by the informing unit, the control unit switches the substitution-fluid supply line from the one of the removal-side substitution-fluid supply line and the return-side substitution-fluid supply line to the other.

3. The blood purification apparatus according to Claim 1, wherein if it is determined to switch the substitution-fluid supply line from one of the removal-side substitution-fluid supply line and the return-side substitution-fluid supply line to an other, the control unit automatically switches the substitution-fluid supply line from the one of the removal-side substitution-fluid supply line and the return-side substitution-fluid supply line to the other.

4. The blood purification apparatus according to any of Claims 1 to 3, further comprising a clogging-identifying unit configured to identify any clogging position of a blood flow route, wherein if the clogging position is identified at a removal-side position of the blood circuit by the clogging-identifying unit, it is determined to switch from the removal-side substitution-fluid supply line to the return-side substitution-fluid supply line to supply the substitution fluid at the time of blood return.

5. The blood purification apparatus according to Claim 4, wherein the return-side substitution-fluid supply line includes a plurality of lines connected to a return-side part of the blood circuit, wherein the blood purification apparatus further includes a first pressure sensor configured to detect a pressure at a removal-side position of the blood circuit; and a second pressure sensor and a third pressure sensor configured to detect pressures on a removal side and a return side, respectively, of the blood purifier at respective return-side positions of the blood circuit, wherein any clogging position is identified with reference to the pressures detected by the first pressure sensor, the second pressure sensor, and the third pressure sensor, and wherein it is determined to switch from the removal-side substitution-fluid supply line to the return-side substitution-fluid supply line to supply the substitution fluid.

6. The blood purification apparatus according to Claim 5, wherein the return-side substitution-fluid supply line includes a pre-substitution line connected to the removal-side blood circuit and through which the substitution fluid is allowed to be supplied; and a post-substitution line connected to the return-side blood circuit and through which the substitution fluid is allowed to be supplied, and wherein it is determined to switch from the removal-side substitution-fluid supply line to the pre-substitution line or the post-substitution line to supply the substitution fluid.

7. The blood purification apparatus according to Claim 6, further comprising a peristaltic substitution pump configured to supply the substitution fluid to the blood circuit through the pre-substitution line or the post-substitution line.

8. A blood purification apparatus comprising:
a blood circuit including a removal-side blood circuit for removing blood from a patient and a return-side blood circuit for returning the blood to the patient, the blood of the patient being caused to extracorporeally circulate through the blood circuit with a blood purifier being connected to the removal-side blood circuit and to the return-side blood circuit, the blood purifier being configured to purify the blood;
a blood pump provided to the blood circuit and configured to cause the blood of the patient to extracorporeally circulate through the blood circuit;
a removal-side substitution-fluid supply line connected to the blood circuit and through which substitution fluid is allowed to be supplied to the blood circuit at a time of blood return;
a return-side substitution-fluid supply line connected to a position of the blood circuit or to the blood purifier and through which the substitution fluid is allowed to be supplied to the blood circuit at the time of blood return, the position of the blood circuit being on a return side relative to a part to which the removal-side substitution-fluid supply line is connected;
a pressure sensor configured to detect a pressure in a flow route at a specific position of the blood circuit;
a flow-rate-detecting unit configured to detect a flow rate in a flow route at a specific position of the blood circuit; and
a clogging-position-identifying unit configured to identify, by using a learned model, any clogging position with reference to the pressure change and the flow rate detected by the pressure sensor and the flow-rate-detecting unit, the learned model being obtained through machine learning in which a plurality of pieces of data on pressure changes and flow rates at occurrences of clogging or narrowing of the flow route in the blood circuit are accumulated to generate cumulative data to be used as teaching data for identifying the clogging position of the flow route.

9. The blood purification apparatus according to Claim 8, wherein the clogging-position-identifying unit identifies, by using a learned model, the clogging position with reference to the pressure change and the flow rate detected by the pressure sensor and the flow-rate-detecting unit and determines to switch to an appropriate one of the substitution-fluid supply lines at the identification of the clogging position, the learned model being obtained through machine learning in which not only a plurality of pieces of data on pressure changes and flow rates at occurrences of clogging or narrowing of the flow route in the blood circuit but also a plurality of pieces of data representing switching to an appropriate one of the substitution-fluid supply lines at the identification of the clogging position are accumulated to generate cumulative data to be used as teaching data for identifying the clogging position and for switching to the appropriate one of the substitution-fluid supply lines at the identification of the clogging position.

10. The blood purification apparatus according to Claim 9, further comprising:
an execution unit configured to switch the substitution-fluid supply line from one of the removal-side substitution-fluid supply line and the return-side substitution-fluid supply line to an other; and
a switching-informing unit configured to provide information to switch to one of the substitution-fluid supply lines that is determined to be appropriate, the information being provided if a clogging position is identified by the clogging-position-identifying unit and it is determined to switch to an appropriate one of the substitution-fluid supply lines.

11. The blood purification apparatus according to Claim 9, further comprising an execution unit configured to switch the substitution-fluid supply line from one of the removal-side substitution-fluid supply line and the return-side substitution-fluid supply line to an other, wherein if a clogging position is identified by the clogging-position-identifying unit and it is determined to switch to an appropriate one of the substitution-fluid supply lines, the execution unit automatically switches to one of the substitution-fluid supply lines that is determined to be appropriate.

12. A blood purification apparatus comprising:
a blood circuit including a removal-side blood circuit for removing blood from a patient and a return-side blood circuit for returning the blood to the patient, the blood of the patient being caused to extracorporeally circulate through the blood circuit with a blood purifier being connected to the removal-side blood circuit and to the return-side blood circuit, the blood purifier being configured to purify the blood;
a blood pump provided to the blood circuit and configured to cause the blood of the patient to extracorporeally circulate through the blood circuit;
a pressure sensor configured to detect a pressure in a flow route at a specific position of the blood circuit;
a flow-rate-detecting unit configured to detect a flow rate in a flow route at a specific position of the blood circuit; and
a clogging-estimating unit configured to estimate, by using a learned model, any clogging position that is to occur in a predetermined time period with reference to the pressure change and the flow rate detected by the pressure sensor and the flow-rate-detecting unit, the learned model being obtained through machine learning in which a plurality of pieces of data on pressure changes and flow rates at occurrences of clogging or narrowing of the flow route in the blood circuit are accumulated to generate cumulative data to be used as teaching data for identifying the clogging position of the flow route.

13. The blood purification apparatus according to Claim 12, wherein the clogging-estimating unit uses teaching data generated from pressure changes and flow rates detected by the pressure sensor and the flow-rate-detecting unit in a process of priming or blood purification treatment, learns whether the pressure changes and the flow rates detected by the pressure sensor and the flow-rate-detecting unit are within predetermined ranges, and estimates the clogging position that is to occur in the predetermined time period with reference to the pressure change and the flow rate detected until a current time by the pressure sensor and the flow-rate-detecting unit.

14. The blood purification apparatus according to Claim 12 or 13, further comprising:
an execution unit configured to start blood return; and
a blood-return-informing unit configured to provide information to start blood return if the clogging position is estimated to occur in the predetermined time period by the clogging-estimating unit.

15. The blood purification apparatus according to Claim 14, wherein if the clogging position is estimated to occur in the predetermined time period by the clogging-estimating unit, the blood-return-informing unit provides information on a remaining time before the flow route causes clogging or narrowing.

16. The blood purification apparatus according to Claim 12 or 13, further comprising an execution unit configured to start blood return, wherein if a clogging position is estimated to occur in the predetermined time period by the clogging-estimating unit, the execution unit automatically starts blood return.
